# EUROPEAN PATENT APPLICATION

(11) **EP 4 772 026 A1**
(43) Date of publication of application: **08.07.2026**
(21) Application number: 24859913.6
(22) Date of filing: 29.08.2024
(51) Int. Cl.: A23L 33/135, A23C 9/00, A61K 35/745, A61P 1/00, A61P 1/10, A61P 1/12, A61P 25/20, A61P 25/22, A61P 25/24, C12N 1/20, C12N 15/09

(54) **SEROTONIN PRODUCTION PROMOTING COMPOSITION**

(30) Priority: 31.08.2023 JP 2023141717
(71) Applicant: Morinaga Milk Industry Co., Ltd., Minato-ku Tokyo 105-7122 (JP)
(72) Inventor: AOKI, Takahiro, Zama-shi, Kanagawa 252-8583 (JP); YOSHIMOTO, Shin, Zama-shi, Kanagawa 252-8583 (JP)
(74) Representative: Vossius & Partner Patentanwälte Rechtsanwälte mbB
(86) International application number: PCT/JP2024/030999
(87) International publication number: WO 2025/047886

(57) **Abstract**

The serotonin production-promoting composition contains one kind or more selected from a bacterium which is *Bifidobacterium bifidum* MCC2085 (NITE BP-03881), a culture of the bacterium and a processed product of the bacterium. The serotonin production-promoting composition contains one kind or more selected from a bacterium which is *Bifidobacterium longum* subsp. *infantis* M-63 (NITE BP-02623), a culture of the bacterium and a processed product of the bacterium.

## Description

### Technical Field

The present invention relates to a serotonin production-promoting composition.

The present application claims priority from patent application No. 2023-141717 filed in Japan on August 31, 2023, and the contents thereof are incorporated here by reference.

### Background Art

Serotonin is one of neurotransmitters in the brain and is involved in physiological functions such as neuroendocrine secretion, sleep, and modulation of body temperature. In addition, serotonin also has a function of controlling the secretion of dopamine and noradrenalin for mental stabilization. Serotonin is biologically synthesized from tryptophan, which is an essential amino acid, through 5-hydroxytryptophan and is distributed in the raphe nuclei of the hypothalamus, the basal ganglia and the medulla oblongata and the like.

It is known that serotonin in the brain is generally produced in the raphe nuclei of the medulla oblongata. NPL 1 discloses the possibility that serotonin produced in the small intestine involves signaling through the vagal spine and affects the brain.

Moreover, in recent years, many studies for the purpose of inhibiting onset of diseases or promoting health or another purpose by actively taking bacteria which positively influence animals (so-called good bacteria, also called "probiotics") and regulating the enteric environment have been conducted. For example, PTL 1 discloses probiotics which have an immunomodulatory effect and which are useful for treating a lesion related to a change in the immune system.

### Citation List

### Patent Literature

PTL 1: JP2013-521335A

### Non Patent Literature

NPL 1: Martin et al., "The Brain-Gut-Microbiome Axis", Cellular and Molecular Gastroenterology Hepatology, Vol. 6, No. 2, pp. 133-148, 2018

### Summary of Invention

### Technical Problem

Metabolism by the intestinal microbiota and the interaction of the intestines and the intestinal microbiota are believed to affect the production of serotonin in the intestines.

A problem of the invention is to provide a serotonin production-promoting composition containing a bacterium of *Bifidobacterium.*

### Solution to Problem

The invention includes the following aspects.
[1] A serotonin production-promoting composition containing one kind or more selected from a bacterium which is *Bifidobacterium bifidum* MCC2085 (NITE BP-03881), a culture of the bacterium and a processed product of the bacterium.
[2] The serotonin production-promoting composition according to [1] which is a food or drink composition.
[3] The serotonin production-promoting composition according to [2] in which the food or drink composition is a probiotic composition or a nutritional composition.
[4] The serotonin production-promoting composition according to [3] in which the nutritional composition is a formula.
[5] The serotonin production-promoting composition according to [3] or [4], further containing a human milk oligosaccharide.
[6] The serotonin production-promoting composition according to [1] which is a pharmaceutical composition.
[7] A serotonin production-promoting composition containing one kind or more selected from a bacterium which is *Bifidobacterium longum* subsp. *infantis* M-63 (NITE BP-02623), a culture of the bacterium and a processed product of the bacterium.
[8] The serotonin production-promoting composition according to [7] in which the serotonin-producing composition is a food or drink composition.
[9] The serotonin production-promoting composition according to [8] in which the food or drink composition is a probiotic composition or a nutritional composition.
[10] The serotonin production-promoting composition according to [9] in which the nutritional composition is a formula.
[11] The serotonin production-promoting composition according to [9] or [10] which further contains a human milk oligosaccharide.
[12] The serotonin production-promoting composition according to [7] which is a pharmaceutical composition.

As another aspect, the invention includes the following embodiments.
[13] A method for promoting serotonin production including administering a bacterium which is *Bifidobacterium bifidum* MCC2085 (NITE BP-03881) to a subject of administration at an intake of 1×10³ to 1×10¹² CFU/day.
[14] A method for promoting serotonin production including administering a bacterium which is *Bifidobacterium infantis* M-63 (NITE BP-02623) to a subject of administration at an intake of 1×10³ to 1×10¹² CFU/day.
[15] Use of a bacterium which is *Bifidobacterium bifidum* MCC2085 (NITE BP-03881) for the promotion of serotonin production.
[16] Use of a bacterium which is *Bifidobacterium infantis* M-63 (NITE BP-02623) for the promotion of serotonin production.

### Advantageous Effects of Invention

According to the aspects, a serotonin production-promoting composition containing a bacterium of *Bifidobacterium* can be provided.

### Brief Description of Drawings

[FIG. 1] FIG. 1 is a graph showing the serotonin production amount of MCC2085.
[FIG. 2] FIG. 2 is a graph showing the serotonin production amount of M-63.
[FIG. 3] FIG. 3 is a graph showing the Tphl mRNA amount of MCC2085.
[FIG. 4] FIG. 4 is a graph showing the Tph1 mRNA amount of M-63.

### Description of Embodiments

A preferable embodiment for carrying out the invention is explained below. In this regard, the embodiment explained below shows an example of typical embodiments of the present disclosure, and the scope of the present technique is not construed as being narrower by the embodiment. In the present specification, a numerical range expressed with "a lower limit to an upper limit" means that the lower limit and the upper limit are included.

In the present specification, when the *"Bifidobacterium bifidum* MCC2085 (NITE BP-03881, also simply referred to as "MCC2085" below)" content of the composition is explained, the content can also be applied to the *Bifidobacterium longum* subsp. *infantis* M-63 (NITE BP-02623, also simply referred to as "M-63" below) content of the composition. When the composition contains both MCC2085 and M-63, the content of MCC2085 alone can be applied to the total content of MCC2085 and M-63.

In the present specification, the "CFU" means Colony Forming Unit. In the present specification, when MCC2085 or M-63 is sterilized cells, the CFU can be replaced with the "cells".

*Bifidobacterium* spp. have been reported to have various physiological functions. It has been reported that proliferation in the intestines or the substances produced by *Bifidobacterium* spp. (for example, acetic acid) are involved in the physiological functions. Accordingly, the bacteria of *Bifidobacterium* of the embodiment can also be expected to be highly safe and show the generally known efficacies of *Bifidobacterium* spp. in a wide age group. In addition, the bacteria of *Bifidobacterium* of the embodiment can be used for the purpose of promoting serotonin production.

### <Bacteria of Bifidobacterium>

*Bifidobacterium bifidum* MCC2085 (NITE BP-03881) is novel *Bifidobacterium bifidum* isolated from feces of a human infant and was deposited for an international deposit as a novel bacterial strain on April 13, 2023 to NITE Patent Microorganisms Depositary (NPMD), National Institute of Technology and Evaluation (address: #122, 2-5-8 Kazusakamatari, Kisarazu-shi, Chiba 292-0818, Japan) as *Bifidobacterium bifidum* MCC2085 (accession number: NITE BP-03881). The bacterium can be generally acquired from the preservation organization.

*Bifidobacterium longum* subsp. *infantis* M-63 (NITE BP-02623) was deposited for an international deposit on January 26, 2018 to NITE Patent Microorganisms Depositary (NPMD), National Institute of Technology and Evaluation (address: #122, 2-5-8 Kazusakamatari, Kisarazu-shi, Chiba 292-0818, Japan) as *Bifidobacterium infantis* M-63 (accession number: NITE BP-02623). The bacterium can be generally acquired from the preservation organization.

MCC2085 and M-63 are not limited to the bacteria themselves deposited and registered at a predetermined organization under the bacterial names (also called "deposited bacteria" below for the convenience of explanation) but include a substantially equivalent bacterium thereof (also called "derived strain" or "induced strain"). Regarding the bacteria, a "substantially equivalent bacterium of a deposited bacterium" means a bacterium which belongs to the identical species to that of the deposited bacterium and which has an equivalent or better feature of the present technique to that of the deposited bacterium of this case. The substantially equivalent bacterium of a deposited bacterium may be, for example, a derived strain of the deposited bacterium. The derived strain is a bacterium bred from the deposited bacterium or a bacterium naturally generated from the deposited bacterium.

In the embodiment, the substantially equivalent bacterium of MCC2085 or M-63 is, for example, a bacterium of the same genus in which the base sequence of 16S rRNA gene has a homology of preferably 99.9% or more, more preferably 100% to the base sequence of 16S rRNA gene of MCC2085 or M-63 and which preferably has the identical bacteriological properties to those of MCC2085.

In the embodiment, MCC2085 or M-63 includes a mutant of MCC2085 or M-63 as long as the mutant has the feature, namely the feature that the serotonin production-promoting effect is high. Moreover, the mutant is preferably a bacterium which has the identical bacteriological properties to those of MCC2085 or M-63 and which has a high serotonin production-promoting effect. Whether a mutant has a "high serotonin production-promoting effect" which is equivalent to that of MCC2085 can be examined using a bacterium cultured by the method described in (2) Preparation of Bacterial Cell-Containing Solutions of the Test Examples described below by the methods described in (4) Quantification of Serotonin Production Amount and (5) Examination of Expression of Tph1 Gene.

Such a mutant may be constructed by introduction of a non-artificial mutation to MCC2085 or M-63. Moreover, such a mutant may be constructed by introduction of a mutation to the bacterium through treatment using a mutagen such as UV or constructed by introduction of a mutation to the bacterium through various genetic engineering methods.

Examples of the substantially equivalent bacterium and the derived strain include the bacteria below.
(1) A bacterium which is identified as the identical bacterium by the Randomly Amplified Polymorphic DNA method or the Pulsed-field gel electrophoresis method (described in Probiotics in food/Health and nutritional properties and guidelines for evaluation 85 Page 43)
(2) A bacterium which possesses the deposited bacterium-derived genes only and does not have any exogenous genes and which has a DNA identity of 95% or more (preferably 98% or more)
(3) A bacterium having the identical characteristics to those of a bacterium bred from the bacterium (including genetic engineering modification, mutation or spontaneous mutation)

MCC2085 and M-63 can be proliferated by culturing the bacteria. The culture method is not particularly limited as long as MCC2085 and M-63 can be proliferated, and a method which is generally used for culturing *Bifidobacterium* spp. can be used with appropriate modification as necessary. For example, the culture temperature may be 30 to 50°C and is preferably 35 to 45°C. MCC2085 and M-63 are preferably cultured under an anaerobic condition and can be cultured, for example, while sending an anaerobic gas such as carbon dioxide gas. Furthermore, MCC2085 and M-63 may be cultured under a microaerophilic condition such as static liquid culture.

The culture medium for culturing MCC2085 and M-63 for proliferation is not particularly limited, and a culture medium which is generally used for culturing *Bifidobacterium* spp. can be used with appropriate modification as necessary. For example, as a carbon source, saccharides such as galactose, glucose, fructose, mannose, cellobiose, maltose, lactose, sucrose, trehalose, starch hydrolysate and molasses can be used. As a nitrogen source, for example, ammonia and ammonium salts and nitrates such as ammonium chloride and ammonium nitrate can be used. Moreover, as an inorganic salt, for example, sodium chloride, potassium chloride, potassium sulfate, magnesium sulfate, calcium chloride, calcium nitrate, manganese chloride, and ferrous sulfate can be used. Furthermore, organic components such as soybean powder, a defatted soybean cake, meat extract and yeast extract may also be used. As a modified culture medium, for example, MRS culture medium can be preferably used.

### <Serotonin Production-Promoting Composition>

The serotonin production-promoting composition of the embodiment (also simply referred to as "composition" below) contains one kind or more selected from a bacterium which is *Bifidobacterium bifidum* MCC2085 (NITE BP-03881), a culture of the bacterium and a processed product of the bacterium. As another aspect, the serotonin production-promoting composition of the embodiment contains one kind or more selected from a bacterium which is *Bifidobacterium longum* subsp. *infantis* M-63 (NITE BP-02623), a culture of the bacterium and a processed product of the bacterium. The serotonin production-promoting composition of the embodiment may contain both MCC2085 and M-63. The serotonin production-promoting composition includes a food or drink composition, a pharmaceutical composition and the like.

In the present specification, whether "the serotonin production-promoting effect is high" can be examined using a bacterium obtained by culturing the bacterium of *Bifidobacterium* by the method described in (2) Preparation of Bacterial Cell-Containing Solutions of the Test Examples described below by the methods described in (4) Quantification of Serotonin Production Amount and (5) Examination of Expression of Tph1 Gene. Specifically, when the ratio of the serotonin production amount of the test bacterial cells to the serotonin production amount of the control is 1.5 or more, preferably 2 or more, more preferably 2.5 or more in (4) Quantification of Serotonin Production Amount, it can be considered that the "serotonin production-promoting effect is high". Moreover, when the ratio of the expression level of Tph1 mRNA of the test bacterial cells to the expression level of Tph1 mRNA of the control is 1.5 or more, preferably 2 or more, more preferably 2.5 or more in (5) Examination of Expression of Tph1 Gene, it can be considered that the "serotonin production-promoting effect is high".

As the bacteria which are MCC2085 and M-63, MCC2085 and M-63 described above can be used, and bacterial cells alone can be collected from the culture medium after culturing and used.

As the cultures of the bacteria, an obtained culture may be used directly after culture. Alternatively, a diluted or concentrated culture may be used, or a culture supernatant collected from a culture may also be used. That is, examples of the cultures of the bacteria include a culture itself, a diluted culture, a concentrated culture, and a culture supernatant.

As long as the effects of the invention are exhibited, bacterial cells of MCC2085 and M-63 which have been subjected to additional treatment such as heating, drying, disruption and sterilization after culture can also be used. The treatments can be used appropriately in combination.

The drying method may be a spray drying method or a lyophilization method. The sterilization method may be a retort sterilization method, a UHT (Ultra High Temperature) sterilization method, a pressurized sterilization method, a high-pressure steam sterilization method, a dry heat sterilization method, a flow steam sterilization method, an electromagnetic wave sterilization method, an electron beam sterilization method, a high-frequency sterilization method, a radiation sterilization method, a ultraviolet sterilization method, an ethylene oxide gas sterilization method, a hydrogen peroxide gas plasma sterilization method, a chemical sterilization method (specifically, an alcohol sterilization method, a formalin fixation method, and an electrolyzed water treatment method) or the like.

That is, the processed products of the bacteria are not particularly limited, and examples thereof include a bacterial cell concentrate, lyophilized bacterial cells, spray dried bacterial cells, sterilized bacterial cells, heat sterilized bacterial cells, and a disrupted bacterial cells.

MCC2085 and M-63 are derived from a human and thus have fewer side effects and are highly safe, and thus an animal can take MCC2085 and M-63 continuously for a long time. Thus, MCC2085 and M-63 can be used for products for a wide variety of uses such as a food or drink composition and a pharmaceutical composition and can be used after mixing with a general carrier or diluent or the like which is acceptable physiologically or pharmaceutically or in terms of foods and drinks.

When an animal takes the composition of the embodiment, a serotonin production-promoting effect can be expected to be obtained in the body.

The MCC2085 content of the entire composition of the embodiment is not particularly limited and may be, for example, 10³ CFU/g or mL or more, 10⁴ CFU/g or mL or more, 10⁵ CFU/g or mL or more, or 10⁶ CFU/g or mL or more, and may be 10¹² CFU/g or mL or less, 10¹¹ CFU/g or mL or less, or 10¹⁰ CFU/g or mL or less.

The MCC2085 or M-63 content of the composition of the invention may be 10³ CFU/g or mL to 10¹² CFU/g or mL, 10⁴ CFU/g or mL to 10¹¹ CFU/g or mL, or 10⁵ CFU/g or mL or more to 10¹⁰ CFU/g or mL.

When an animal takes the composition of the embodiment, a serotonin production-promoting effect can be expected to be obtained in the body. The composition of the invention can be used for preventing, improving or treating a symptom or a disease caused by lack of serotonin.

Examples of the symptom or the disease caused by lack of serotonin include a symptom or a disease caused by stress, a symptom or a disease caused by a disorder of the intestinal microbiota, and premenstrual syndrome.

Examples of the symptom or the disease caused by stress include neuropsychiatric symptoms (for example, headaches, giddiness, slight fever, anxiety, depression, melancholic feeling, decreased motivation or concentration, insecurity, feeling of despair, irritation and dyssomnia) and general symptoms (for example, lassitude and malaise).

Examples of the symptom or the disease caused by a disorder of the intestinal microbiota include diarrhea, constipation, and abdominal pain.

Examples of the premenstrual syndrome include vasomotor symptoms (for example, palpitation and swelling), neuropsychiatric symptoms (for example, headaches, giddiness, slight fever, anxiety, depression and dyssomnia), locomotor organ symptoms (for example, lower back pain and joint pain), digestive system symptoms (for example, constipation, diarrhea, abdominal pain, increased appetite and thirstiness), general symptoms (for example, lassitude and malaise) and skin symptoms (for example, acne).

An example of the anxiety is anxiety disorder. The anxiety disorder is a generic term for symptoms causing strong anxiety and behavioral or mental disorder and means, for example, panic disorder, obsessive-compulsive disorder, and social anxiety disorder (performance anxiety).

Examples of the depression include melancholic feeling, decreased motivation or concentration, insecurity, malaise, feeling of despair, irritation, dyssomnia, and autonomic disorder.

Examples of the dyssomnia include sleeping disorder, sleeplessness and poor sleep. The sleeping disorder is a generic term for symptoms or diseases related to sleep, and examples thereof include insomnia, and hypersomnia.

That is, the composition of the embodiment can be used as an anti-stress composition, an anxiolytic composition, an anti-depressive composition, a sleep aid composition, an intestinal microbiota-improving composition, an intestinal regulation composition and a premenstrual syndrome-improving composition.

Thus, in the present specification, a therapeutic use and a non-therapeutic use are provided. The therapeutic use includes especially treatment or prevention of a disease caused by lack of serotonin. For example, treatment or prevention of a disease selected from the group consisting of headaches, giddiness, slight fever, anxiety, depression, depressive feeling, anxiety, insomnia, sadness, panic disorder, premenstrual syndrome (PMS), irritability, diarrhea, constipation, abdominal pain, palpitation, tumefaction, lower back pain, joint pain, acne, obsessive-compulsive disorder, social anxiety disorder and hypersomnia is expected.

The non-therapeutic use includes especially prevention of a physical or mental condition related to lack of serotonin. Such a condition is not always desirable. In particular, improvement of any one or a plurality of conditions selected from the group consisting of decreased motivation or concentration, insufficient sleep, fatigue, malaise, increased appetite, thirst and autonomic dysfunction is expected.

Serotonin is one of neurotransmitters and is found widely in the central nerve system and the entire blood circulation system. Recent studies suggest that serotonin is an essential chemical substance in neural development such as cell division, neuronal migration, cell differentiation, synapse formation and synaptic plasticity. Furthermore, it has also been found that serotonin regulates synapses and thus favorably affects speech, memory, motility and emotional learning. It has also been found in recent studies that serotonin involves the peristaltic movement of the intestines and promotes the motility of the stomach and the digestion process. From these facts, it is also believed that promotion of serotonin production in the body may be effective also for the above diseases.

The medical use and the non-medical use provided in the present specification are particularly advantageous for desired medical or non-medical intervention in an infant and/or a child, preferably a human infant and/or a human child.

The composition containing MCC2085 may be used for a therapeutic purpose or used for a non-therapeutic purpose.

The "non-therapeutic purpose" is a concept which does not include medical practice, namely treatment of a human body by therapy. Examples thereof include health promotion and beauty treatment.

The "improvement" means improvement of a disease, a symptom or the condition; prevention or delay of deterioration of a disease, a symptom or the condition; or reversal, prevention, or delay of progress of a disease or a symptom.

The "prevention" means prevention or delay of the onset of a disease or a symptom in the subject of the application or reduction of the risk of a disease or a symptom of the subject of the application.

When the composition of the invention is used for a non-therapeutic purpose, the composition can be administered to a healthy individual as the subject. The healthy individual may be a subject without any symptom or disease caused by lack of serotonin. When the composition of the invention is used for a non-therapeutic purpose, the composition can be used for further promoting serotonin production in the body of a healthy individual.

When the composition of the invention is used for a non-therapeutic purpose, the composition can promote serotonin production in a healthy individual and can prevent or improve a symptom caused by stress, anxiety, depression, dyssomnia, a symptom caused by a disorder of the intestinal microbiota, premenstrual syndrome, or the like.

When the composition of the invention is used for a therapeutic purpose, the composition can be administered to a non-healthy individual as the subject. An example of the non-healthy individual is a subject having a symptom or a disease caused by lack of serotonin.

When the composition of the invention is used for a therapeutic purpose, the composition can promote serotonin production in a non-healthy individual and can prevent, improve or treat a symptom or a disease caused by stress, anxiety, depression, dyssomnia, a symptom or a disease caused by a disorder of the intestinal microbiota, premenstrual syndrome, or the like.

The serotonin production-promoting composition containing MCC2085 or M-63 may be used for a human or a nonhuman animal (preferably a mammal) as the subject of application, preferably for a human or a pet, more preferably for a human. Furthermore, the subject to which the present technique is applied is not particularly limited as long as the subject wishes for a probiotic effect, and examples thereof include an infant, a young child, a child, an adult, a healthy individual, a middle-aged individual, an aged individual, and those with poor enteric environment.

In this manner, MCC2085 and M-63 can also be contained in a probiotic composition as an active ingredient. Moreover, MCC2085 and M-63 can also be used for the manufacture of the compositions. Furthermore, MCC2085 and M-63 can also be used as a bacterium for probiotic.

Regarding the administration or the intake of MCC2085 or M-63, MCC2085 or M-63 is preferably taken continuously for at least one week or more preferably taken continuously for at least four weeks. During the administration or intake period of MCC2085 or M-63, MCC2085 or M-63 is desirably taken every day.

The used amount of MCC2085 or M-63 is not particularly limited because of the high safety and, for example, the amount is preferably 1×10⁵ to 1×10¹² CFU/kg body weight/day, more preferably 1×10⁷ to 1×10¹¹ CFU/kg body weight/day, further preferably 1×10⁸ to 1×10¹⁰ CFU/kg body weight/day. Alternatively, the used amount per one individual (body weight) (in other words, dose) is preferably 10⁷ to 10¹⁴ CFU/day, more preferably 10⁸ to 10¹³ CFU/day, further preferably 10⁹ to 10¹² CFU/day.

Moreover, the used amount of MCC2085 or M-63 is not particularly limited because of the high safety, and the dry weight of the bacterial cells is preferably 0.01 to 10000 mg/body weight kg/day, more preferably 0.01 to 1000 mg/body weight kg/day, still further preferably 0.01 to 100 mg/body weight kg/day.

The used amount of MCC2085 or M-63 is not particularly limited because of the high safety, and is preferably 0.001 to 100 mL/body weight kg/day, more preferably 0.1 to 100 mL/body weight kg/day.

### <Pharmaceutical Composition>

The pharmaceutical composition which is the serotonin production-promoting composition of the embodiment is not particularly limited as long as the pharmaceutical composition contains MCC2085 or M-63. The pharmaceutical composition may contain both MCC2085 and M-63.

The pharmaceutical composition of the embodiment is not particularly limited as long as MCC2085 and M-63 themselves, a culture of the bacteria or a processed product of the bacteria is contained.

The pharmaceutical composition of the embodiment contains MCC2085 or M-63, which have been obtained from the human intestines, as an active ingredient and thus can be administered as an oral composition component without worries also to a patient having various diseases. Moreover, because the bacteria of *Bifidobacterium* also exist in the intestines of animals, it is expected that the embodiment does not easily cause side effects even when the embodiment is administered to an animal continuously for a long time. In addition, the bacteria of *Bifidobacterium* can be administered safely also to an infant, a young child or a child. Thus, the embodiment is also preferable for preventing, improving and treating a disease or a symptom thereof of an infant, a young child or a child.

The use of the pharmaceutical composition of the embodiment is for a non-healthy individual as a subject, and the pharmaceutical composition is used for the therapeutic purpose above.

The dosage form of the pharmaceutical composition of the embodiment is not particularly limited. Specifically, examples of the dosage form of the pharmaceutical composition include tablets, pills, powder, liquid preparation, suspensions, emulsions, granules, capsules, syrups, suppositories, injections, ointment, patches, eye drops, and nasal drops. Moreover, for the formulation, an additive which is generally used as a carrier for formulation, such as excipients, binders, disintegrating agents, lubricants, stabilizers, flavoring agents, diluents, surfactants and solvents for injection, can be used.

For the formulation, a component which is generally used for formulation, such as excipients, pH-adjusting agents, colorants and corrigents, can be used for the pharmaceutical composition according to the embodiment. As long as the effects of the invention are not impaired, a component having an effect of preventing, improving and/or treating a disease or a symptom which is known or will be found in the future and which is relevant to the embodiment can also be used for the pharmaceutical composition according to the embodiment. In addition, the formulation can be appropriately conducted by a known method depending on the dosage form. The formulation may also be conducted by appropriately blending a carrier for formulation.

The MCC2085 or M-63 content of the pharmaceutical composition of the embodiment is appropriately determined based on the dosage form, the usage, the age of the patient, the gender, the type of disease, the degree of the disease, the other conditions and the like, and is generally preferably in the range of 1×10⁵ to 1×10¹² CFU/g or 1×10⁵ to 1×10¹² CFU/mL, more preferably in the range of 1×10⁷ to 1×10¹¹ CFU/g or 1×10⁷ to 1×10¹¹ CFU/mL.

The timing of administration of the pharmaceutical composition of the embodiment is not particularly limited, and the timing of administration can be appropriately selected according to the method for treating the subject symptom or disease. The pharmaceutical composition may be administered prophylactically or used for a maintenance therapy. Moreover, the form of administration is preferably determined based on the formulation form, the age of the patient, the gender, the other conditions, the degree of the symptom of the patient, or the like. In this regard, in all the cases, the pharmaceutical composition of the embodiment can be administered once a day or in separate portions and may be administered once in several days or in several weeks.

### <Food or Drink Composition>

The food or drink composition of the embodiment is not particularly limited as long as one kind or more selected from MCC2085 or M-63 itself, cultures of the bacteria and processed products of the bacteria are contained, and may be a probiotic composition, a nutritional composition or a general food or drink composition.

The form of the food or drink composition of the embodiment is not particularly limited and may be a powder, granules, a paste, an emulsion, oil drops, capsules, tablets or the like.

Regarding the use of the food or drink composition of the embodiment, the food or drink composition is used for any subject including a healthy individual and a non-healthy individual and is used for the non-therapeutic purpose above.

The food or drink composition of the embodiment may be produced by adding one kind or more selected from MCC2085 or M-63 itself, cultures of the bacteria and processed products of the bacteria to a known food or a known drink, or a new food or drink composition can be produced by mixing the same into a raw material of a food or a drink. The food or drink composition may contain both MCC2085 and M-63.

Examples of a general food or drink composition include: drinks such as soft drinks, carbonated drinks, nutritional drinks, fruit juices and lactic acid bacteria beverages (including concentrated undiluted solutions of the drinks and powders for preparation thereof); frozen desserts such as ice creams, sherbets and shaved ice; confectioneries such as sweets, candies, gums, chocolates, tablet candies, snacks, biscuits, jellies, jams, creams and baked sweets; dairy products such as processed milk, milk beverages, fermented milk, drink yogurts and butter; and breads. Moreover, the food or the drink may be a supplement and may be, for example, a supplement in the tablet form. In the case of a supplement, MCC2085 or M-63 can be taken while the amount of meals and the calorie intake per day are not affected by other foods.

Moreover, the food or drink composition in the embodiment may be a probiotic composition containing a component which has a probiotic effect or a component which supplements a probiotic effect as long as the effects of the invention are not impaired. For example, the food or drink composition in the embodiment can be prepared by combining MCC2085 or M-63 with a component such as: carbohydrates such as human milk oligosaccharides; proteins such as whey protein, casein protein, soybean protein and pea protein or mixtures or decomposition products thereof; amino acids such as leucine, valine, isoleucine and glutamine; vitamins such as vitamin B6 and vitamin C; creatine; citric acid; and fish oil.

Moreover, the food or drink composition defined in the embodiment can be provided or sold as a food or a drink with a label with use of probiotics or the like (including a health use). Moreover, the food or the drink can be provided or sold with a label for "those who want to increase serotonin in the body", "those who want to be in a cheerful mood", "those who want to become strong against stress", "those who want to improve uneasy feeling", "those who want to improve diarrhea", "those who want to improve constipation", "those who want to improve premenstrual syndrome", "those who sleep poorly", "those who want to have a sound sleep", "those who wish to lead a life with *Bifidobacterium* spp.", "those who want to improve the enteric environment", "those who want to correct the stomach condition", "those who want to form a good enteric environment", "those who are bothered by premenstrual syndrome" or the like as the subject of intake. The "labeling" act includes all the acts for informing a consumer of the use, and all the expressions which can remind of or cause to guess the use are the "labeling" acts of the invention, regardless of the purposes of labeling, the contents of labeling, the objects to be labeled, the media, and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery, or importing such an article, an act of displaying or distributing an advertisement of a product, a price list or a business document with a description of the use thereon or providing information with such contents with a description of the use by an electromagnetic method (specifically the Internet or the like), and another act.

The content of the label is preferably a label approved by the administration or the like (for example, a label approved based on various systems provided by the administration and provided in the form based on the approval). It is preferable to label with such a content on packaging, a container, a catalogue, a brochure, an advertisement material in a sales site such as POP (Point of Purchase) advertisement, other documents or the like.

The "labels" also include labels with health foods, functional foods, enteral nutrition products, foods for special dietary uses, foods with health claims, foods for specified health uses, foods with nutrient function claims, foods with function claims and the like. In particular, exemplified are labels approved by the Consumer Affairs Agency in Japan, such as labels approved by the systems for foods for specified health uses, foods with nutrient function claims or foods with function claims, or a similar system. Specific examples include a label with foods for specified health uses, a label with qualified foods for specified health uses, a label indicating influence on the structure or the function of a body, a label with reduction of disease risk, and a label with a scientifically grounded function. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The MCC2085 or M-63 content of the food or drink composition of the embodiment is appropriately determined based on the form of the food or drink composition, and the content of the food or the drink is generally preferably in the range of 1×10⁶ to 1×10¹² CFU/g or 1×10⁶ to 1×10¹² CFU/mL, more preferably in the range of 1×10⁷ to 1×10¹¹ CFU/g or 1×10⁷ to 1×10¹¹ CFU/mL.

The food or drink composition according to the embodiment can be used as a nutritional composition. In the embodiment, the "nutritional composition" means a food or a drink which is orally taken mainly for the purpose of taking nutrients. The kind of the nutritional composition which can be used in the embodiment is not particularly limited, and is preferably a formula, liquid food (enteral nutrition products, porridge or the like), baby food, a sport drink or the like, more preferably a formula. The subject of intake may be an infant, a young child, a child or an adult, and is preferably an infant or a young child.

The formulas include a powdered formula and a liquid formula. In the present specification, the powdered formula is defined as "those obtained by processing raw milk derived from cow, raw goat's milk, raw sheep's milk, cow's milk, certified milk, or a food produced therefrom as a raw material or using such a material as a major raw material, adding nutrients necessary for infants and small children and processing into powder". In the present specification, the liquid formula is defined as "those obtained by processing raw milk derived from cow, raw goat's milk, raw sheep's milk, cow's milk, certified milk, or a food produced therefrom as a raw material or using such a material as a major raw material, adding nutrients necessary for infants and small children and processing into liquid".

The formula of the embodiment may further contain a human milk oligosaccharide. The human milk oligosaccharide is not particularly limited as long as it is an oligosaccharide generally contained in human milk, and preferable examples thereof include neutral human milk oligosaccharides such as 2'-fucosyllactose, 3-fucosyllactose, lactodifucotetraose, 2',3-difucosyllactose, lacto-N-triose II, lacto-N-tetraose, lacto-N-neotetraose, lacto-N-fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose and lacto-N-neohexaose, and acidic human milk oligosaccharides such as 3'-sialyllactose, 6'-sialyllactose, 3-fucosyl-3'-sialyllactose and disialyl-lacto-N-tetraose. Of these, 2'-fucosyllactose, 3-fucosyllactose, 3'-sialyllactose and 6'-sialyllactose are preferable, and 2'-fucosyllactose is particularly preferable.

The formulas also include those which contain nutrient components such as various proteins, oils and fats, carbohydrates, minerals and vitamins. As the nutrient component, the nutrient components described above can be used. The formulas further include "a powdered infant formula", "a liquid infant formula" and "a powdered formula for pregnant and parturient women and nursing mothers" of the food for special dietary uses provided by the Health Promotion Act and also include aspects such as a powdered formula for infants and small children for infants of the age of 0 to 12 months, follow-up milk for infants and younger children of the age of 6 to 9 months or older (until the age of 3 years), a powdered formula for low birthweight infants for newborns with a body weight at birth less than 2500 g (low birthweight infants), a powdered formula for an allergy used and a powdered formula for lactose intolerance for treating infants and small children with sickness such as milk allergy and lactose intolerance, a powdered formula for inborn errors of metabolism, a powdered formula for small children, nutritional powder for adults, and nutritional powder for the aged.

The formula of the embodiment can be administered to any subject including a healthy individual and a non-healthy individual.

When the formula of the embodiment is administered to a healthy individual, the formula is used for a powdered infant formula, follow-up milk, a powdered formula for low birthweight infants, a powdered formula for small children, nutritional powder for adults or nutritional powder for the aged and can be used for the non-therapeutic purpose above.

When the formula of the embodiment is administered to a non-healthy individual, the formula is used for a powdered formula for an allergy, a powdered formula for lactose intolerance or a powdered formula for inborn errors of metabolism and can be used for the therapeutic purpose above.

The food or drink composition containing a nutritional composition according to the embodiment can be applied to food with health claims or food for the ill. The system for food with health claims has been established not only for general foods but also for foods in the form of tablets, capsules or the like, in view of the trends inside and outside of Japan and also considering the consistency with the existing system for foods for specified health uses. The system includes two types, namely foods for specified health uses which are of the individual approval system and foods with nutrient function claims which are of the standard regulation system.

### <Production Method of Serotonin Production-Promoting Composition>

The method for producing a serotonin production-promoting composition of the embodiment is explained below. In the method for producing a serotonin production-promoting composition of the embodiment, the step of adding MCC2085 or M-63 may be conducted at any stage in the production step of the composition.

The method for producing a serotonin production-promoting composition includes at least a step of mixing MCC2085 or M-63 and a raw material. Examples of the raw material include the raw material of the serotonin production-promoting composition described above, a component which has a probiotic effect, a component which supplements a probiotic effect, and a carrier for formulation. In the case of a method for producing a composition for a food or a drink, at least the raw material of the food or drink composition described above is contained as the raw material. In the case of a method for producing a composition for a food or a drink, at least one kind of a component which has a probiotic effect, a component which supplements a probiotic effect, a carrier for formulation and the like may be contained as the raw material.

When MCC2085 or M-63 and a raw material are mixed, MCC2085 or M-63 may be bacterial cells, a culture containing bacterial cells or a processed product containing bacterial cells. The form of the bacterial cells or the culture containing bacterial cells is not particularly limited and may be liquid or solid, but the form is preferably a dried form (for example, a bacterial powder and a dried culture) because handling during the production and during the storage is easy. Moreover, the culture may be, as described above, bacterial cells themselves from which the culture solution has been separated, a mixture of bacterial cells and a culture solution, a culture solution from which bacterial cells have been removed or the like. The product means a component that MCC2085 produces when MCC2085 or M-63 is cultured.

When MCC2085 or M-63 and the raw material are mixed, MCC2085 or M-63 and the raw material are preferably mixed in such a manner that the MCC2085 or M-63 content based on the total mass of the composition becomes 1×10³ to 1×10¹² CFU/g, further preferably 1×10⁵ to 1×10¹¹ CFU/g.

The method for producing a composition may further include a step of drying a mixture of MCC2085 or M-63 and the raw material. The drying method is not particularly limited as long as the effects of the embodiment are not impaired. Specific drying methods include a spray drying method, a retort sterilization method, lyophilization method, a UHT sterilization method, a pressurized sterilization method, a high-pressure steam sterilization method, a dry heat sterilization method, a flow steam sterilization method, an electromagnetic wave sterilization method, an electron beam sterilization method, a high-frequency sterilization method, a radiation sterilization method, a ultraviolet sterilization method, an ethylene oxide gas sterilization method, a hydrogen peroxide gas plasma sterilization method, a chemical sterilization method (specifically, an alcohol sterilization method, a formalin fixation method, and an electrolyzed water treatment method), and the like. In the embodiment, a lyophilization method or a spray drying method is preferable.

The method for producing a composition may include at least one of a step of further mixing a prebiotic in the mixture and a step of further mixing a milk component in the mixture.

The method for producing a composition may further include a step of mixing at least one kind of bacterium of probiotics such as *Bifidobacterium* strains other than MCC2085 or M-63 and lactic acid bacteria.

The milk component is preferably a powder and is more preferably mixed with a bacterial powder of MCC2085 or M-63.

The milk component is not particularly limited as long as the effects of the embodiment are not impaired. Examples of the milk component include cow's milk, buffalo's milk, sheep's milk, goat's milk, mare milk, skim milk, concentrated skim milk, skim milk powder, concentrated milk, whole milk powder, cream, butter, buttermilk, condensed milk, and a milk protein. One kind or two or more kinds selected from the group consisting thereof can be used. The milk protein is not particularly limited, and examples thereof include whey, casein, and hydrolysates thereof. One kind or two or more kinds selected from the group consisting thereof can be used. The milk component is preferably a component derived from cow's milk.

Regarding the hydrolysates, a whey hydrolysate, a casein hydrolysate or the like can be produced by hydrolyzing the milk component (preferably a milk protein). Examples of the hydrolysis include acid or alkali hydrolysis and enzymatic hydrolysis. Of these, enzymatic hydrolysis is preferable. The enzyme used for enzymatic hydrolysis is preferably a proteolytic enzyme. Examples of the proteolytic enzyme include protease, trypsin, chymotrypsin, plasmin, pepsin, papain, peptidase, and aminopeptidase. The pH during the hydrolysis reaction is appropriately adjusted to the optimum pH of the enzyme used, and the hydrolysis reaction can be conducted, for example, at pH 2 to 6. The temperature during the hydrolysis reaction is not particularly limited and is generally preferably in the range of 30 to 60°C. The reaction period is preferably two to 10 hours.

When a supplement or tablets are produced as the composition of the invention, the method for producing a composition of the embodiment can include a step of mixing a culture containing MCC2085 or M-63 and a carrier for formulation or the like and thus obtaining a mixture and a step of forming into the predetermined shape of the supplement or the tablets. The forming step is, for example, tableting. An example of tableting is obtaining tablets by compression molding of a powder or a granulated mixture.

Furthermore, an example of the method for producing a composition of the embodiment is a method for producing a fermented food or drink (preferably a fermented milk) containing MCC2085 or M-63. A step of adding a dried mixture of MCC2085 or M-63 and the raw material to a raw fermented milk material and thus obtaining a fermented milk containing MCC2085 or M-63 is conducted. Alternatively, a step of mixing a dried mixture of MCC2085 or M-63 and the raw material and a fermented milk and thus obtaining a fermented milk containing MCC2085 or M-63 is conducted. The fermentation condition and the blending amount are adjusted in such a manner that the bacterial cell amount of MCC2085 or M-63 in the composition becomes the bacterial cell amount described above. As a result, a fermented food or drink, preferably a fermented milk or a fermented product, containing MCC2085 or M-63 can be provided.

### <Method for Promoting Serotonin Production and Method for Preventing and Improving Disease or Symptom Caused by Lack of Serotonin>

The method for promoting serotonin production of the invention includes administering a bacterium which is *Bifidobacterium bifidum* MCC2085 (NITE BP-03881) or *Bifidobacterium infantis* M-63 (NITE BP-02623) to a subject of administration at an intake of 1×10³ to 1×10¹² CFU/day.

Because MCC2085 or M-63 promotes serotonin production, when MCC2085 or M-63 is administered to a subject of administration who expects for at least one of anti-stress, anti-anxiety, anti-depression, sleep improvement, improvement of a symptom caused by a disorder of the intestinal microbiota and improvement of premenstrual syndrome or a subject of administration feeling a problem with the intestines at an intake of 1×10³ to 1×10¹² CFU/day, the symptom can be expected to be improved. The intake of MCC2085 or M-63 by the subject of administration is preferably conducted continuously.

That is, MCC2085 or M-63 can be used for at least one of anti-stress, anti-anxiety, anti-depression, sleep improvement, improvement of a symptom caused by a disorder of the intestinal microbiota and improvement of premenstrual syndrome of a subject of administration. Moreover, a bacterium which is *Bifidobacterium infantis* M-63 (NITE BP-02623) can be used particularly for at least one of sleep improvement, improvement of a symptom caused by a disorder of the intestinal microbiota and improvement of premenstrual syndrome.

For example, the sleep-improving method of the invention includes administering a bacterium which is MCC2085 or M-63 to a subject of administration at an intake of 1×10³ to 1×10¹² CFU/day. Similarly, the anxiety-improving method, the depression-improving method, a method for improving a symptom caused by a disorder of the intestinal microbiota and a method for improving premenstrual syndrome of the invention include administering a bacterium which is MCC2085 or M-63 to a subject of administration at an intake of 1×10³ to 1×10¹² CFU/day.

### Examples

The embodiment is explained in further detail below based on Examples. In this regard, the Examples explained below show examples of typical Examples of the embodiment, and the scope of the invention is not construed as being narrower due to the Examples.

### <Test Examples>

### (1) Object

The Test Example was conducted for the purpose of quantifying the serotonin production amounts and examining the expression of Tph1 gene of *Bifidobacterium bifidum* MCC2085 (NITE BP-03881) and *Bifidobacterium infantis* M-63 (NITE BP-02623).

### (2) Preparation of Bacterial Cell-Containing Solutions

MRS culture medium (Difco^{™} Lactobacill MRS Broth, type number 288130) was sterilized at 121°C for 15 minutes. MCC2085 or M-63 was cultured in the MRS culture medium after sterilization under an anaerobic condition at 37°C for 16 hours. After the culture solutions after the culture were centrifuged to collect the respective bacterial cells, the collected bacterial cells were suspended in phosphate buffered saline (PBS) and adjusted to 1×10⁹ cells/ml, and thus an MCC2085-containing solution and an M-63-containing solution were obtained.

### (3) Culture and Seeding of RIN14B Cells

RIN14B cells (ATCC CRL-2059, rat pancreatic cancer-derived enterochromaffin-like cells) were cultured in an RPMI 1640 culture medium containing 10% fetal bovine serum (FBS) and 1% penicillin-streptomycin (manufactured by FUJIFILM Wako Pure Chemical Corporation) under a condition at 37°C and 5% CO₂ and subcultured once in three to four days.

The RIN14B cells after the culture were incubated using 0.25 w/v% trypsin/EDTA (gibco) at 37°C for five minutes and diluted using the RPMI 1640 culture medium containing 10% FBS and 1% penicillin-streptomycin, and thus a solution containing 2.5×10⁵ cells/mL of RIN14B cells was obtained. The RIN14B cell-containing solution was seeded in a 24-well plate at 500 µL/one well, and then the cells were further cultured under a condition at 37°C and 5% CO₂ for 48 hours.

### (4) Quantification of Serotonin Production Amount

The RIN14B cells after culturing in (3) above were washed once with an HBSS(+) solution (FUJIFILM Wako Pure Chemical Corporation). The MCC2085- or M-63-containing solutions prepared in (2) above were diluted 20-fold using an RPMI 1640 culture medium containing 1% penicillin-streptomycin and added to the wells at 500 µL/one well, and the cells were cultured under a condition at 37°C and 5% CO₂ for 24 hours. After culturing for 24 hours, the culture supernatants were collected, and the serotonin contents of the culture supernatants were measured using an ELISA kit (manufactured by DLD, type number EA602/96).

Here, the same procedures as those above were conducted except that the bacterial cell-containing solutions were not added to obtain a control.

### (5) Examination of Expression of Tph1 Gene

After culturing in (4) above, the RIN14B cells remaining in the wells were collected using Trizol (manufactured by Thermo Fisher Scientific), and RNAs were extracted. cDNAs were synthesized from the extracted RNAs using PrimeScript^{™} RT Master Mix (Perfect Real Time), and then qRT-PCR (quantitative Real-time PCR) was conducted using takara TB GreenR Premiz Ex Taq (registered trademark) II (Tli RNaseH Plus).

As shown in Table 1, for Tph1 gene, the values measured using Tphl F primer (SEQ ID NO: 1) and Tph1 R primer (SEQ ID NO: 2) were normalized with the expression level of GAPDH mRNA measured using Rat GAPDH F primer (SEQ ID NO: 3) and Rat GAPDH R primer (SEQ ID NO: 4).

**[Table 1]**

| Primer | Sequence |
|---|---|
| *Tph1* F | AACAAAGACCATTCCTCCGAAAG |
| *Tph1* R | TGTAACAGGCTCACATGATTCTC |
| Rat GAPDH F | ACATTGTTGCCATCAACGAC |
| Rat GAPDH R | CTTGCCGTGGGTAGCGTCAT |

### (6) Results

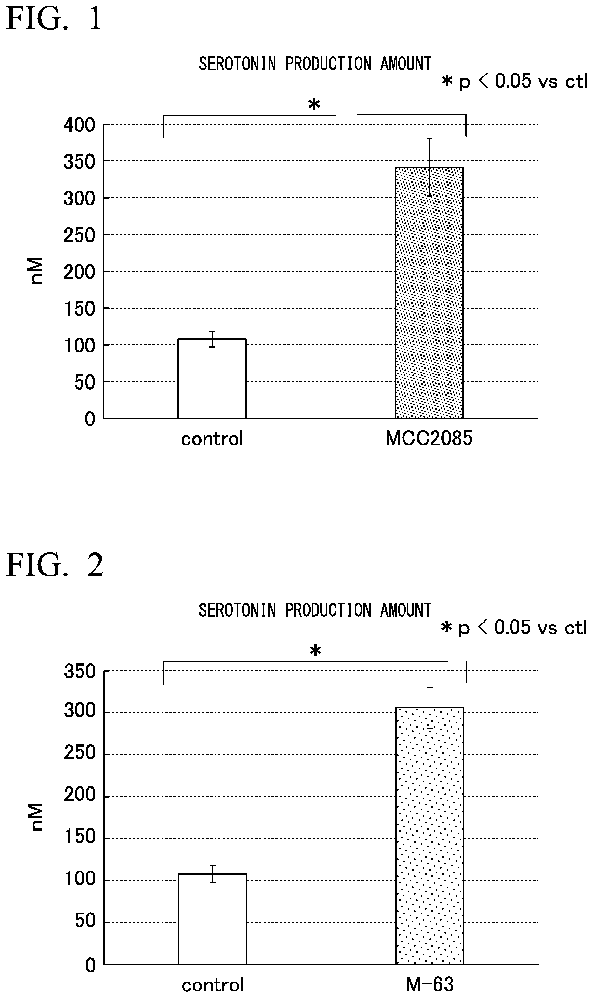

FIG. 1 is a graph showing the serotonin production amount in MCC2085. FIG. 2 is a graph showing the serotonin production amount in M-63. For comparison between two groups of the control and MCC2085 or M-63, t-test was used, and statistical analysis was conducted.

Compared to the control without the addition of the bacterial cells, the serotonin production amount increased with significance when MCC2085 was added and when M-63 was added. The ratios of the serotonin production amounts of the bacteria MCC2085 and M-63 to the serotonin production amount of the control were 3.2 and 2.8, respectively.

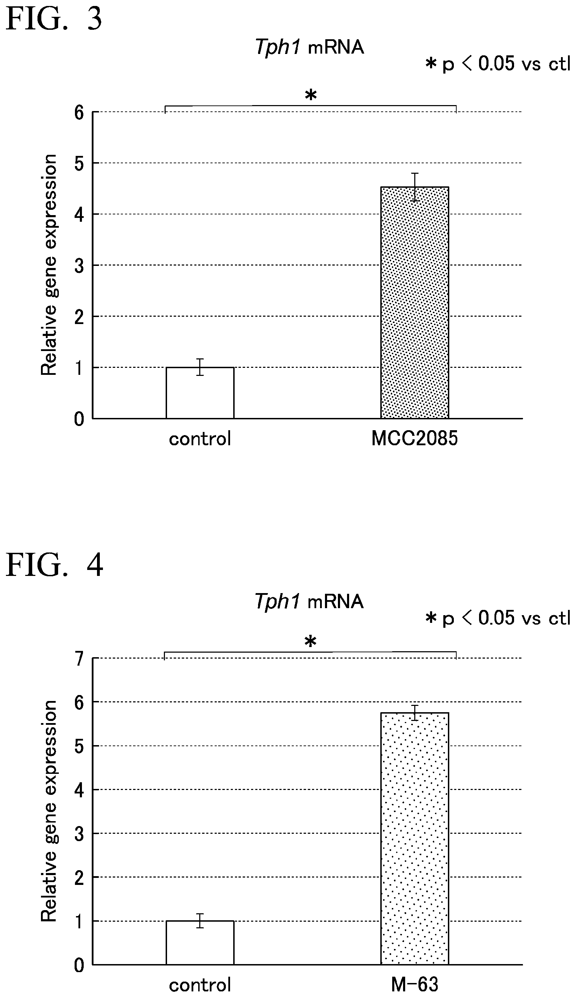

FIG. 3 is a graph showing the relative expression level of Tph1 mRNA in MCC2085. FIG. 4 is a graph showing the relative expression level of Tph1 mRNA in M-63. For comparison between two groups of the control and MCC2085 or M-63, t-test was used, and statistical analysis was conducted.

As shown in FIGs. 3 and 4, compared to the control without the addition of the bacterial cells, the mRNA expression level increased with significance when MCC2085 was added and when M-63 was added. The ratios of the expression levels of Tph1 mRNA of the bacteria MCC2085 and M-63 to the expression level of Tph1 mRNA of the control were 4.5 and 5.7, respectively.

Thus, it was found that MCC2085 and M-63 have an ability to produce serotonin.

### [Production Examples]

Production Examples of the composition, the pharmaceutical composition, the fermented milk, the powdered formula and the food or drink composition of the embodiment are shown below, but the composition of the embodiment is not limited thereto.

### [Production Example 1]

MCC2085 or M-63 is added to 3 mL of an MRS liquid culture medium (Difco^{™} Lactobacill MRS Broth, type number 288130) and anaerobically cultured at 32 to 39°C for five to 25 hours, and then the culture solution is concentrated and lyophilized to obtain lyophilized powder of the bacterium (namely bacterial powder). The bacterial powder and whey protein concentrate (WPC) are mixed uniformly, and thus a composition is obtained. The composition in an amount of 20 g is dissolved in 200 g of water, and thus a serotonin production-promoting composition containing MCC2085 or M-63 is obtained.

### [Production Example 2]

MCC2085 or M-63 is added to 3 mL of an MRS liquid culture medium and anaerobically cultured at 32 to 39°C for five to 25 hours, and then the culture solution is concentrated and lyophilized to obtain lyophilized powder of the bacterium (namely bacterial powder). The bacterial powder and dry powder of milk protein concentrate (MPC480, manufactured by Fonterra Co-operative Group Limited, protein content of 80 mass%, casein protein:whey protein = about 8:2) are mixed uniformly, and thus a composition is obtained. The composition in an amount of 20 g is dissolved in 200 g of water, and thus a serotonin production-promoting composition containing MCC2085 or M-63 is obtained.

### [Production Example 3]

MCC2085 or M-63 is added to 3 mL of an MRS liquid culture medium and anaerobically cultured at 32 to 39°C for five to 25 hours, and then the culture solution is concentrated and lyophilized to obtain lyophilized powder of the bacterium containing MCC2085 or M-63 (namely bacterial powder). Next, the bacterial powder and crystalline cellulose are introduced into an agitation granulator and mixed. Then, purified water is added for granulation, and the granules are dried to obtain granules containing an excipient (a pharmaceutical composition). In this manner, granules containing MCC2085 or M-63 can be obtained.

### [Production Example 4]

A production method of a fermented milk containing MCC2085 or M-63 is shown below.

First, a raw milk material, water according to the need, other components and the like are mixed, preferably homogenized and heat sterilized. The homogenization and the heat sterilization can be conducted by general methods. A lactic acid bacterium starter is added (namely seeded) to the heat sterilized prepared milk solution, and the solution is kept at a predetermined fermentation temperature and fermented to obtain a fermented product. Through fermentation, curd is formed.

As the lactic acid bacterium starter, for example, lactic acid bacteria which are generally used for yogurt production, such as *Lactobacillus bulgaricus, Lactococcus lactis* and *Streptococcus thermophilus,* can be used. When the pH reaches the target value, the formed curd is crushed by stirring and cooled to 10°C or lower, and a fermented product is thus obtained. By cooling to 10°C or lower, the activity of the lactic acid bacterium can be reduced, and the production of an acid can be suppressed.

Next, the fermented product obtained by the fermentation step is heat treated, and a fermented product after the heating is thus obtained. By appropriately heating the fermented product, the production of an acid by the lactic acid bacterium in the fermented product after the heating can be inhibited. Thus, a decrease in pH during the subsequent production step and/or during the storage of the concentrated fermented milk containing the strain of *Bifidobacterium* can be inhibited. As a result, the viability of the strain of *Bifidobacterium* can be improved.

Next, MCC2085 or M-63 is added to the fermented product after the heating obtained by the heat treatment step. The amount of MCC2085 or M-63 added, relative to the fermented product after the heating, is preferably 1×10⁷ to 1×10¹¹ CFU/mL, more preferably 1×10⁸ to 1×10¹⁰ CFU/mL.

After adding MCC2085 or M-63 to the fermented product after the heating, the mixture is concentrated. The concentration step can be conducted appropriately using a known concentration method. For example, centrifugation method or membrane separation method can be used. Through the centrifugation method, the whey in the product to be concentrated (namely, the fermented product after the heating to which MCC2085 or M-63 has been added) is removed, and a concentrated fermented milk which has an increased solid content concentration is obtained.

A fermented milk containing the obtained MCC2085 or M-63 can be produced as described above.

### [Production Example 5]

A production method of a powdered formula containing MCC2085 or M-63 is shown below.

In 300 kg of warm water, 10 kg of desalted cow's milk whey protein powder (manufactured by MILEI GmbH), 6 kg of cow's milk casein powder (manufactured by Fonterra Cooperative Group Limited), 48 kg of lactose (manufactured by MILEI GmbH), 920 g of a mineral mixture (manufactured by Tomita Pharmaceutical Co., Ltd.), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) were dissolved and further heated and dissolved at 90°C for 10 minutes, and after adding 28 kg of modified fats (manufactured by Taiyo Yushi Corp.), the mixture was homogenized. Then, after sterilization and concentration steps, the mixture was spray dried, and about 95 kg of a powdered formula was thus prepared. To the powdered formula, 100 g of bacterial powder of MCC2085 or M-63 (1.8×10¹¹ CFU/g, manufactured by Morinaga Milk Industry Co., Ltd.) triturated in starch is added, and about 95 kg of a powdered formula containing the strain of *Bifidobacterium* and oligosaccharides is thus prepared. When the obtained powdered formula is dissolved in water and a formula solution having a total solid content concentration as a standard formula concentration of 14% (w/V) is obtained, 10⁶ to 10⁹ CFU/100 mL can be obtained as the *Bifidobacterium* strain count in the formula solution.

By taking or administering the powdered formula containing MCC2085 or M-63 obtained in the above manner, the enteric environment of a human can be improved.

### [Production Example 6]

A production method of a food for improving enteric environment containing MCC2085 or M-63 is shown below.

MCC2085 or M-63 is added to 3 mL of an MRS liquid culture medium and anaerobically cultured at 32 to 39°C for five to 25 hours, and then the culture solution is concentrated and lyophilized to obtain lyophilized powder of the bacterium (namely bacterial powder) and to thus obtain a composition of the embodiment. The bacterial powder of the embodiment can be added to a food containing a large amount of cellulose (for example, vegetables or salad) and cooked, and a composition of the embodiment can also be thus obtained.

Alternatively, the bacterial powder is taken at a meal, before a meal, during a meal or after a meal. Because dietary fibers are automatically taken with a regular diet (a diet with staple food, a side dish and a main dish), an effect of improving the enteric environment of an adult can be expected when the dry bacterial powder product of the embodiment is taken together during a meal.

When the food for improving enteric environment of the embodiment is continuously taken by a person feeling a problem with the intestines, such as constipation and diarrhea, a higher effect of improving enteric environment can be expected.

Therefore, a serotonin production-promoting composition containing *Bifidobacterium bifidum* MCC2085 (NITE BP-03881) or *Bifidobacterium infantis* M-63 (NITE BP-02623) can promote serotonin production.

## Claims

1. A serotonin production-promoting composition comprising one kind or more selected from a bacterium which is *Bifidobacterium bifidum* MCC2085 (NITE BP-03881), a culture of the bacterium and a processed product of the bacterium.

2. The serotonin production-promoting composition according to claim 1 which is a food or drink composition.

3. The serotonin production-promoting composition according to claim 2, wherein the food or drink composition is a probiotic composition or a nutritional composition.

4. The serotonin production-promoting composition according to claim 3, wherein the nutritional composition is a formula.

5. The serotonin production-promoting composition according to claim 3 or 4, further comprising a human milk oligosaccharide.

6. The serotonin production-promoting composition according to claim 1 which is a pharmaceutical composition.

7. A serotonin production-promoting composition comprising one kind or more selected from a bacterium which is *Bifidobacterium longum* subsp. *infantis* M-63 (NITE BP-02623), a culture of the bacterium and a processed product of the bacterium.

8. The serotonin production-promoting composition according to claim 1 which is a food or drink composition.

9. The serotonin production-promoting composition according to claim 8, wherein the food or drink composition is a probiotic composition or a nutritional composition.

10. The serotonin production-promoting composition according to claim 9, wherein the nutritional composition is a formula.

11. The serotonin production-promoting composition according to claim 8 or 9, further comprising a human milk oligosaccharide.

12. The serotonin production-promoting composition according to claim 1 which is a pharmaceutical composition.
